# EUROPEAN PATENT APPLICATION

(11) **EP 4 488 254 A1**
(43) Date of publication of application: **08.01.2025**
(21) Application number: 23183635.4
(22) Date of filing: 05.07.2023
(51) Int. Cl.: C07C 29/153, C07C 29/157, C07C 31/04, B01J 31/20

(54) **PROCESS FOR PRODUCING METHANOL**

(71) Applicant: C1 Green Chemicals AG, 12489 Berlin (DE)
(72) Inventor: Checinski, Marek Pawel, 12487 Berlin (DE); Clauss, Reike, 12527 Berlin (DE); Akash, Kaithal, 12524 Berlin (DE); Suntrup, Lisa, 10559 Berlin (DE)
(74) Representative: Keil & Schaafhausen Patentanwälte PartGmbB

(57) **Abstract**

The invention is directed to process for catalysing a production of methanol from hydrogen and carbon monoxide. The reaction takes place in the presence of at least one nucleophilic promoter, whereby a complex is used which comprises a transition metal (M) and at least one ligand. The ligand is designed as a chelating agent that binds tightly to three or more sites with two donor atoms which are connected via a ligand backbone with at least one Lewis base centrum (LB). The invention covers two cases 1 and 2. In case 1, both donor atoms are phosphor atoms whereby each donor atom has two identical substituents R' or R" each and whereby the substituents R' and R" differ from each other. In case 2, at least one of the donor atoms is a phosphor atom which is a chiral center with two different substituents R' and R".

## Description

The invention is directed to process for catalyzing a production of methanol from hydrogen and carbon monoxide taking place in the presence of at least one nucleophilic promoter, whereby a complex is used which comprises a transition metal (M) and at least one ligand designed as a chelating agent that binds tightly to three or more sites with two donor atoms which are connected via a ligand backbone with at least one Lewis base centrum (LB).

Methanol, also known as CH₃OH or MeOH (a methyl group linked to a hydroxyl group), is a chemical compound that plays a crucial role in the chemical industry. It serves not only as a fuel component but also as a fundamental building block in various chemical processes. In 2018, its production volume exceeded 100 million metric tons.

However, a drawback of all known reaction mechanisms is that they require harsh conditions, particularly temperatures above 250 °C and high pressures exceeding 100 bar. This results in energy-intensive processes, which pose challenges from both economic and environmental perspectives. To address this, reducing energy consumption can lead to cost savings and enhance the sustainability of the process by reducing operational expenses (opex) and capital expenses (capex).

The invention aims to provide a process for producing methanol with significantly lower energy requirements compared to the processes known from the state of the art. This is important from both an economic and environmental perspective, as it can reduce costs and improve the sustainability of the process. By reducing energy consumption, the process can lower opex and capex, resulting in cost savings. Additionally, the reduced energy requirements contribute to a more sustainable and environmentally friendly production of methanol.

From document WO 2020/136003 A1 it is already known to use pincer type complexes as a catalyst. However, only homogeneous ligand structures are used which means that in cases a pincer type ligand is used, each type of donor atoms of the pincer type ligand features only one type of substitute. This holds particularly true for P atoms as donor atoms which are complete substituted with either isopropyl or phenyl rests. The use of at least two different substituents for one type of donor atoms and the correlation between these types and the donor atoms is not described.

However, the invention aims to optimize the production of methanol by developing a process that is more energy-efficient, cost-effective, and environmentally sustainable compared to existing methods.

Surprisingly, it has been found that in contrast to the teaching of WO 2020/136003 A1 at least one phosphor donor atom and the use of at least two different substituents for phosphor donor atoms leads to increased yields which can be proved by new computational simulations and confirmed by experiment, the inventors observed an increase of the turnover rate by a factor of three through the newly designed asymmetric ligand structure for the (pre-) catalysts presented herein. This enables lower reaction temperatures, which leads to lower energy consumption and higher catalyst stability. In addition, at lower temperatures, the reactions are suppressed, so that the selectivity of the reaction is increased. This could be achieved through the specific ligand design, which lowers the activation barrier and/or increases the stability significantly compared to other known catalysts.

Summing up, the invention discloses a process according to the features of claim 1.

In detail, 1 the process takes place in the presence of at least one nucleophilic promoter. Moreover, a complex is used as the basic component for the catalyzed conversion of hydrogen and carbon monoxide. Said complex comprises a transition metal and at least one Lewis basic ligand, preferably one Lewis base ligand designed as a chelating agent that binds tightly to three or more sites including two donor atoms.

The three or more sites binds preferably in adjacent coplanar site, which are most preferably in a meridional configuration. In particularly preferred embodiments, three or four sites are bound. Most preferably, three sides are bound which leads to a pincer type complex. A pincer type complex, including an N atom as the only Lewis base centrum is most preferred as it shows particularly good performance.

Coming back to the two donor atoms, these two donor atoms are connected via a ligand backbone with at least one Lewis base centrum, preferably one or two Lewis base centrum. Both donor atoms can feature substituents each. For the idea underlying the invention two cases 1 and 2 are possible. In case 1 both donor atoms are phosphor atoms whereby each donor atom has two identical substituents R' or R" each and whereby the substituents R' and R" differ from each other. In case 2 at least one donor atom is a phosphor atom and each phosphor atom features a chiral center with two different substituents R' and R". It is essential that there is any kind of asymmetries in the substituents of at least one donor atom which is a phosphor atom.

In this context, the understanding of the wording "ligand backbone" is the molecular connection of the two donor atoms via the at least one Lewis base centrum. Thereby, geometry of the ligand and its binding strength to the metal and reaction partners like H₂, CO, Methanol is affected.

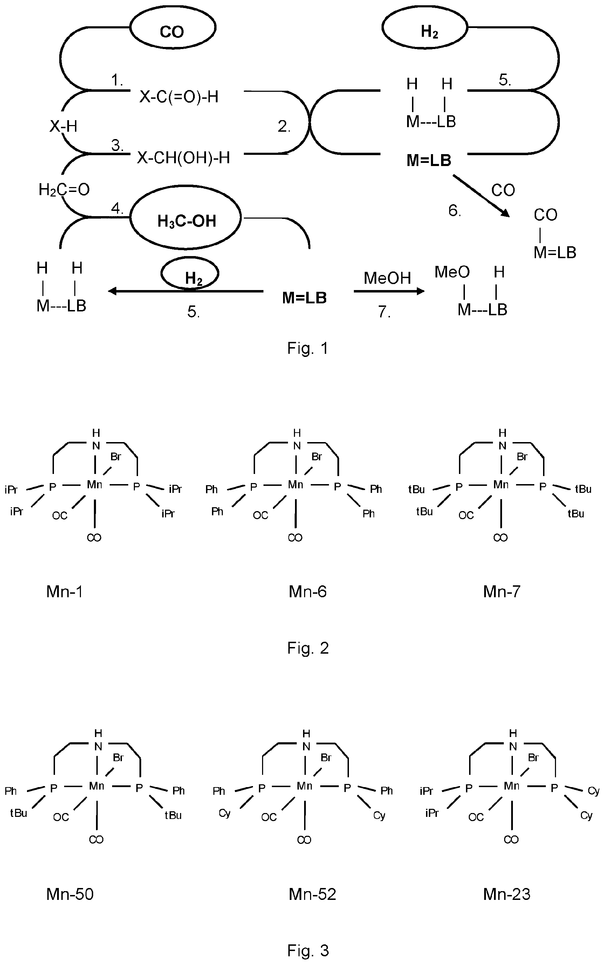

The potential reaction path for the invention as shown in Fig. 1 is described as follows:
1. Base assisted X-H bond split of the promoter (X = N for N-Promoters, and X = O for O-Promoters) and insertion of CO
2. Hydrogenation of HC(X)=O by the catalyst (metal organic complex with asymetric ligand)
3. Elimination of hydrogenation intermediate and followed by a release of formaldehyde and recreation of the promoter X-H.
4. Hydrogenation of formaldehyde to methanol
5. Hydrogenation of M=N to MH-NH.
6. Blocking of active center M=N by CO
7. Blocking of active center M=N by an alcohol, i.e. MeOH

The complex according to the invention is depicted as two forms of an M-LB arrangement, whereby M stand for the transition metal and LB is the center of the at least one Lewis base.

As a starting point, the nucleophilic promoter and CO converts into an intermediate like ester or amide, which will then be hydrogenated by H₂ using the complex according to the invention. The formed intermediate decays into formaldehyde and will be hydrogenated by another H₂ to form the final product methanol in one pot under significant milder conditions than previous catalytic systems. It is essential for the invention that the whole reaction takes part with one mixture and does not require temporally and/or locally separated steps.

The article "Molecularly Defined Manganese Catalyst for Low-Temperature Hydrogenation of Carbon Monoxide to Methanol" published by Pavel Ryabchuk, Kenta Stier, Kathrin Junge, Marek P. Checinski*, and Matthias Beller* in J. Am. Chem. Soc. 2019, 141, 42, 16923-16929 teaches that the symmetric catalyst Mn-1 performs the reaction much better than Mn-6 and Mn-7 (tab. 6). Due to the sterical effect of Mn-7 with its di-tert-Butyl-Phosphine arms leads to close to no conversion. The di-Phenyl-Phosphin arm of Mn-6 shows in comparison to Mn-1 a poor reactivity at synthesis gas conditions as well. The symmetric PNP catalysts Mn-1, Mn-6 and Mn-7 are shown in Fig. 2.

In comparison, the present invention is the first to depart from this doctrine of symmetrical substitution using i.e. the systems Mn-50, Mn-52 and Mn-23 as shown in Fig. 3, which are asymmetric PNP catalysts.

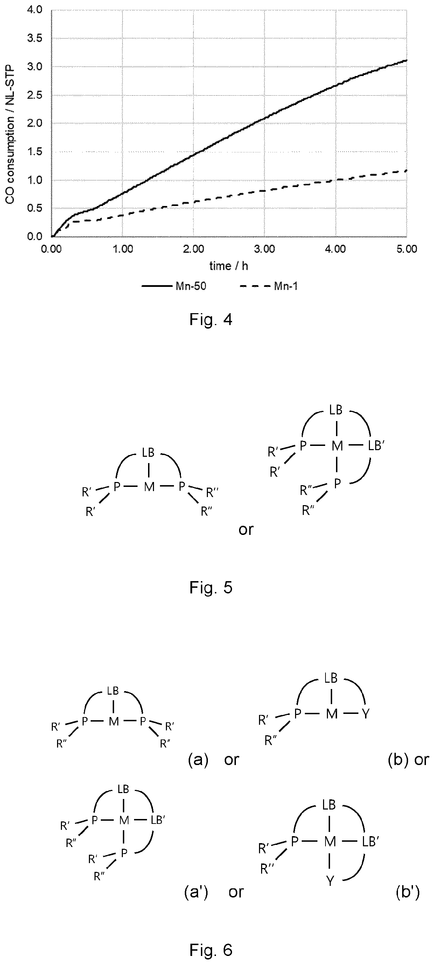

Surprisingly the catalyst Mn-50, which is an asymmetric mixture of the weak performing Ph and tBu rests at the Phosphorus atom, leads to much higher activity than Mn-1 (fig 4).
Fig. 4. shows CO consumption of systems Mn-50 and Mn-1 under identical reaction conditions.

So, it could be confirmed that the asymmetric catalysts like Mn-50 showed lower activation barriers for the rate limiting step (hydrogenation). It shows an improved difference as well between the main reaction 5 of fig. 1 (H₂) and the catalyst deactivation 6 of fig. 1 (CO). The same improvement in respect to Mn-1 could be observed for other asymmetric PNP catalysts like Mn-52 and Mn-23.

**Tab. 1: ΔG° (kJ/mol) values for (pre-)catalysts in this invention compared to reference systems for crucial process steps derived from DFT calculations.**

| **Catalyst** | **TS hydrogenation (step 2)** | **H₂ coordination** | **CO coordination** | **Stability Mn(CO)₅H** |
|---|---|---|---|---|
| Mn-1 (*reference*) | +121 | -33 | -38 | +26 |
| Mn-6 (*reference*) | +112 | -59 | -80 | +27 |
| Mn-7 (*reference*) | +191 | -20 | -18 | +15 |
| Mn-50 | +106 | -59 | -58 | +47 |
| Mn-52 | +108 | -45 | -50 | +43 |
| Mn-23 | +108 | -29 | -21 | +47 |

These results show a significant improvement to classical symmetric PNP catalysts.

Particularly preferred is a system with a metal ion selected from the group manganese (Mn), iron (Fe), ruthenium (Ru), chrome (Cr), molybdenum (Mo), tungsten (W), rhenium (Re), cobalt (Co), rhodium (Rh), iridium (Ir), nickel (Ni) and palladium (Pd) in which manganese, ruthenium, cobalt, or molybdenum in particular exhibit good capabilities. These metals exhibit good coordination of the chelate ligand. In particular, the use of manganese or ruthenium as a central ion shows high turnover rates (TON).

It is preferred that one of the at least one Lewis base center is either an N atom bounded twice to transition metal (M) in form of an amide (N⁻) or an NH group wherein the N atom has a single bound through its electron lone pair to the transition metal (M). Further, in each case two further bond of the N atom are part of the ligand backbone (the donor atoms are each assigned one). Thereby, the intended function of the first Lewis base center is the utilization of the metal ligand cooperative (MLC) effect, where the ligand plays an active part in the reaction mechanism. A metal and ligand of a complex are both involved in the bond breaking or bond formation of a substrate. MLC is most broadly used with M-N systems, but also for example M-NH, M-OH and M-SH metal ligand systems have application in MLC.

In a case a second Lewis Base centrum is foreseen, it is preferred that it is a N (amide), NH, or NR (R = Alkyl, Aryl). First and the second Lewis Base centrum are neighbored. This increases the MLC effect.

Metal organic complexes in the following form are possible, whereby in all shown cases, M is the transition metal, LB the at least one Lewis base centrum positioned in the ligand backbone and coordinated to the transition metal. The substituents R' and R" are not equal to each other. Fig. 5 shows possible (pre-)catalysts with two P donor atoms each showing one type of substituents.

Having a closer look at the first forms shown in fig. 5, both donor atoms are phosphor atoms. Each single donor atom features only one form of substituent R or R", however, the substituents of the two phosphor atoms differ from each other.

However, also, the complexes shown in Fig. 6 are possible, which are possible (pre-)catalysts with at least P donor atom showing two types of substituents.

In the further given forms a, a', b and b', not only two types of substituents are used in view of two donor phosphor atoms, but already at least one donor phosphor atom itself features two different substituents each. In these cases, P-chirality is not limited to specific isomers. Y is defined as a P, N, S or C atom being bound to the Lewis base centrum (LB) and featuring further substituents such that P is specified as PR‴Rʺʺ with R‴ and/or Rʺʺ as ethyl (Et), n-propyl (nPr), isopropyl (iPr), isobutyl (iBu), n-butyl (nBu), t-butyl (tBu), phenyl (Ph), a substituted phenyl (Ph*), naphtyl (Naph) or a substituted naphtyl (Naph*), N is an N atom being part of an amino group (NH₂), a diethylamin (NEt₂), a di-nbutylamin N(nBu)₂ or any type of a pyridine, pyrrole, indol, isoindol, imidazol, benzimidazol or anilin, S is an S atom being part of a thiolate, a methyl sulfide (SMe), a ethyl sulfide (SEt), a phenyl sulfide (SPh), a substituted phenyl sulfide (SPh*), a naphtyl sulfide (SNaph) or a substituted naphtyl sulfide (SNaph*) and C is a C atom being part of an N-heterocyclic carbene, an aryl cyclopentadien or a derivate of cyclopentadien.

It has to be pronounced that for cases with Y including a P atom, it is possible that one or even both substitutions R‴ and Rʺʺ are identical to the substituents R' and R" defined for the other donor atom P. In this case, R‴ and Rʺʺ are not equal. However, in cases R' and R" differ from each other, the substituents R‴ and Rʺʺ can be equal. Further, beside these cases R‴ and/or Rʺʺ can be identical or different to R' and R".

The metal center can require additional co-ligands such as for example, but not limited to, carbon monoxide, halides, nitriles, nitrosyl ligands, N-heterocyclic car-benes or monodentate phosphines.

Having a closer look at the substituents R' or R", at least one of these substituents is preferably an alkyl or an aryl group. Particular good performance can be found if R' and R" is selected from a group comprising ethyl, n-propyl, isopropyl, n-butyl, isobutyl, t-butyl, phenyl, cyclohexyl and naphthyl as well as substituted phenyls, naphthyls and cyclohexyls. Best results are found for configurations wherein both substituent R' and R" are selected from a group comprising ethyl, n-propyl, isopropyl, isobutyl, n-butyl, t-butyl, phenyl, cyclohexyl and naphthyl as well as substituted phenyls naphthyls and cyclohexyls. Nevertheless, R' and R" are two different substituents.

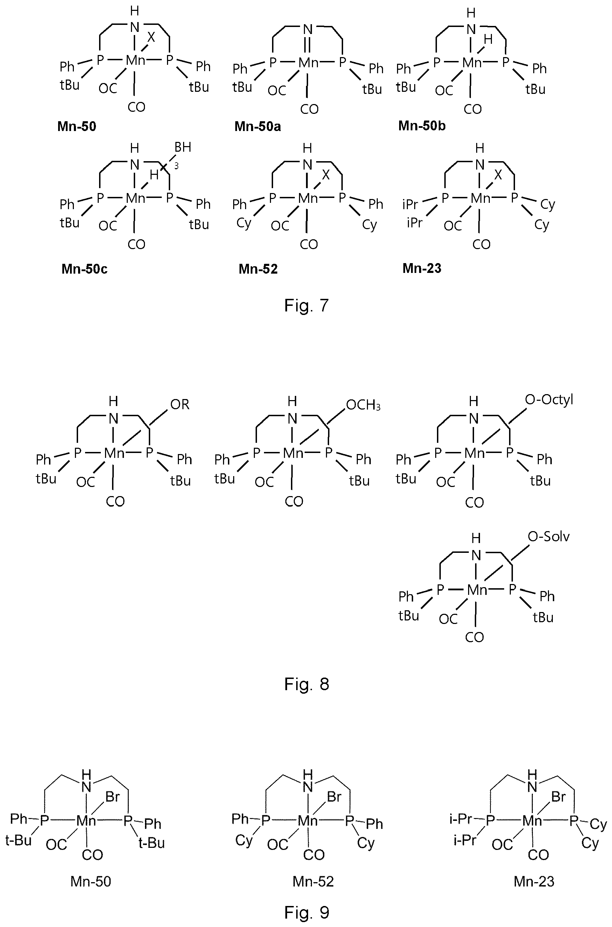

Up to now, the complexes as shown in Fig. 7 are most preferred for the process according to the invention, whereby X is defined as F, Cl, Br, OH or an alcoholate. Starting from the specific example Mn-50b, it is essential to understand that the complex in its catalytic functionality changes its configuration depending on the reaction step. Based on the model shown in figure 1, the possible configurations of the complex Mn-50b as shown in Fig. 8 are present in the solution.

It is also preferred but not limited that the procedure takes place in the presence of a base, which further enables a stabilization of the coordination at the central ion and/or deprotonation. The base is a hydroxide or an alkoxide as it is a well-known and easily available type. The base can act as well as a co-catalyst in the carbonylation step of the promoter.

Additionally, or alternatively, the base is a methoxide salt, tert-butoxide salt or alcoholate of the utilized solvent. This reduces the number of components present in the batch and thus minimizes also possible side reactions.

In particular, it has been found that alkoxide M'-OR of metal ions M' like lithium (Li), sodium (Na), potassium (K), rubidium (Rb), magnesium (Mg), calcium (Ca) or strontium (Sr) can be used to generate formates by direct carbonylation using CO. In this context, a definition of OR as methoxylate, octanolate or O-alkyl leads to good results.

Other bases like boron- (B), aluminum- (Al), titanium- (Ti), zirconium- (Zr), iron-(Fe), manganese- (Mg), cobalt- (Co) or molybdenum (Mo) alkoxides are promising bases as well. These alkoxides can be used in mixtures as well. Upon hydrogenation, the base is regenerated, therefore acting as a co-catalytic agent.

In a preferred embodiment, the promoter contains nitrogen and/or oxygen. As shown in fig. 1, the nucleophilic promoter is essential for capturing CO and transforming it into a formamide or a methyl formate or the respective formate of the alcohol used as solvent.

Furthermore, the applicants combined this group of catalysts with protic, polar solvents, especially primary and/or secondary alcohols as well as mixtures thereof as the reaction medium. Particular preferred are particularly decaline, 1-octanol and/or 2-ethyl-1-hexanol. So the need for an additional promoter is circumvented or at least the amount of promoter is reduced. In addition, these alcohols can advantageously support the catalysis by forming high-boiling formate intermediates (such as octyl formate if octanol was used as solvent; compare Fig. 1, step 1), which can be hydrogenated by the catalyst proposed in this invention. This allows for example to supply with required intermediates (formates or forma-mides) to maximize the reaction rate.

Moreover, particular good turnover numbers can be obtained in cases wherein the ratio r1 between base and catalyst is between 1:1 and 100:1 and/or the ratio r2 of the promoter and base should be between 1:1 and 1:100.

Due to the high catalytic activity of the complexes according to the invention the process is run at a temperature below between 50 and 200 °C, preferably 50 and 150 °C. In addition or alternatively hydrogen as well as carbon monoxide are introduced with a partial pressure between 0.1 and 50 bar.

The process can be performed as a batch, semi-batch process or as a continuous process. This opens a wide range of possibilities in terms of reaction technology.

As a best-case example, process is run at total pressures between 40 and 100 bar and at a temperature between 80 and 150 °C, t the complex has the structural formula 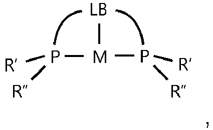 in particular

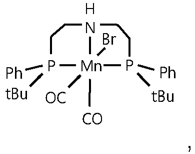

The base is KOMe and/or K-(2-ethyl-1-hexanoate) and the solvent as well as the promotor is 1-octanol and/or 2-ethyl-1-hexanol.

### Improved catalytic performance with new catalyst systems

According to the identified reaction path, the catalysts suggested in this invention were derived from chemical simulations. The most important step of the reaction is the free energy transitions state of the hydrogenation of the substrate (step 2) as depicted in fig.1, as this is the rate determining step in the reaction. Additionally, CO coordination to the active center, rendering the catalyst inaccessible for methanol production, which has to be minimized for maximum turnover rates.

It should be noted that the depicted or suggested structures of catalysts in this invention can in some cases be considered as stable pre-catalysts, which are activated during the reaction to offer a free coordination site for the required hydrogen. The core of the hydrogenation functionality comes from the active center Mn=N_{PNY}, which activates H₂ and converts to the complex Mn(H)-NH_{PNY}. Finally, the Mn(H)-NH_{PNY} transfers 2[H] to formaldehyde and/or formates like methyl formate to form methanol or formates, respectively.

Fig. 9 depicts the molecular structures of exemplary (pre-)catalysts Mn-50, Mn-52 and Mn-23 as possible catalyst representatives of the groups a) and b) according to the invention.

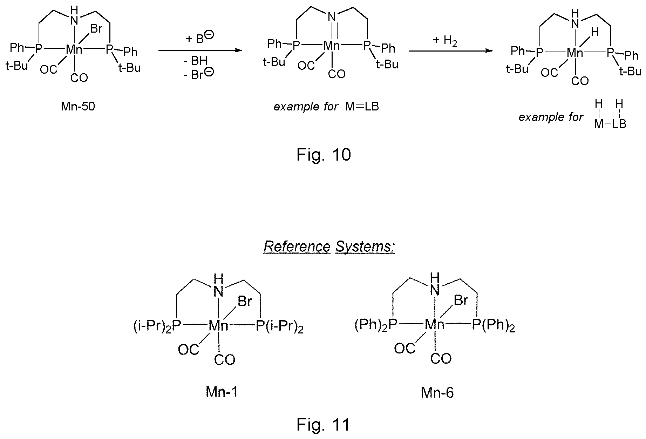

The activation of the (pre-)catalysts towards the active hydrogenation catalyst (compare Fig. 1) can for example be achieved via deprotonation (i.e. through KOMe) of the protic Lewis basic backbone, followed by bromide dissociation, generating a free coordination site for the addition of hydrogen, as shown in Fig. 10 for the new (pre-)catalyst Mn-50 using a base (B⁻) followed by addition of hydrogen.

Other anions or halides than bromine can be used for the (pre-)catalyst as well like, for example, chlorine, BH₄⁻, BHEt₃⁻, OCH₃, octanolate, 2-ethyl-1-hexanoate. The core feature of the catalyst is the Mn=N species, which can be formed by all of these mentioned exemplary pre-catalysts.

Through the flexible incorporation of different substituents on the donor atoms, it is possible to finetune the steric demand at the metal ion to promote substrate coordination or limit blocking of the active site by other reagents in the mixture.

The following examples show individual aspects of the invention. All the features described and/or depicted, either individually or in any combination, constitute the subject matter of the application, irrespective of whether they are depicted in the claims or referred back to them.

### Examples regarding performance of different complex types

To highlight the superiority and impact of the new systems, two reference systems Mn-1 and Mn-6 from the original work in WO 2020/136003 A1 (Fig. 11) bearing symmetrically substituted ligands are used for comparison, as they represent symmetrically substituted systems with either sterically demanding or less demanding substituents.

Chemical simulations (Table 2) of the chosen examples show low free energies for the transition state of the substrate hydrogenation (step 2) as well as for the possible CO coordination as an unfavorable side reaction.

Surprisingly, the new catalysts according to this invention show lower free energies for the hydrogenation transition state, which directly impacts the energy needed to drive the reaction. It is also noteworthy that the systems proposed in this invention show either an improved tendency towards hydrogen coordination compared to the competing, non-productive CO coordination, or lower absolute free energies compared to the reference systems. The combination of both effects yields the improved performance. Additionally, the new catalysts according to the invention show a higher stability regarding the dissociation of the PNP ligand, leading in a syngas atmosphere to the formation of the thermally (above 80 °C, p°) unstable Mn(CO)₅H species, which can then undergo irreversible decomposition of the active species.

Experiments conducted under identical conditions with both the catalysts proposed in this invention as well as with the reference system show an increase of 200% in reaction rate (Fig. 4). The applicants attribute this effect to the lower energy barrier for the rate determining step. This also opens up the possibility to further decrease the reaction temperature while maintaining excellent reaction rates compared to current state-of-the-art commercial applications. This trend is observed under a broad range of conditions.

Furthermore, it is advantageous that the reaction takes place in a polar solvent, particularly alcohols, since alcohols can act as the O-promoters in the reaction, thereby forgoing further additives in the mixture. While many alcohols can be utilized as solvents, 1-octanol and 2-ethyl-1-hexanol are preferred due to their high boiling points to facilitate product isolation by distillation of methanol out of the homogeneous catalyst solution.

The reaction should take place below 200 °C. However, it has proved to be particularly advantageous to carry out the process at a temperature below 150 °C. The temperature of the sample is therefore below 150 °C, even as low as 110 °C. This allows further energy savings and pushes the equilibrium to the product side of the exergonic and endothermic reaction of 2 H₂/CO to methanol. Compared to this, common conditions using the reference system Mn-1 for hydrogenations are utilize temperatures of 150 °C to achieve good turnover rates. Higher temperatures shall be avoided for homogeneous catalyst due to lower catalyst stability or accelerated side reactions.

The total pressure of the reaction can be kept below 100 bar, compared to the traditional industrial process close to 100 bar. CO and H₂ are both introduced into the process preferably at a partial pressure between 0.1 and 50 bar. In particular, CO with a pressure between 0.1 and 25 bar, hydrogen with a pressure between 30 and 50 bar is introduced into the system. Increasing the pressure of the hydrogen slightly above that of the CO ensures that the downstream hydrogenation actually takes place as completely as possible. With this invention, the total pressure of the system can be as low as 40 bar, which is both less energy intensive and safer for handling the reaction. The reaction setup is run in a semi-batch or continuous mode, where the ratio of H₂ to CO in the reactor is larger than 2:1 (stoichiometry for MeOH), and the pressure drop will be compensated by refilling with a gas mixture with a H₂ to CO ratio of 2:1.

Methanol production from CO and H₂ at a total pressure between 10 and 40 bar is therefore particularly commercially preferred, with the pressure of each component being independent of that of the others. The temperature lies between 80 and 150 °C. The catalyst is present in a concentration between 1 and 30 mmol. A Metal-PNY (especially Mn-PNP) complex is used as catalyst. The entire reaction is performed in 1-octanol or 2-ethyl-1-hexanol as O-promoter and solvent. As Base K(OMe) or K(2-ethyl-1-hexanolate) can be used.

### General Procedure for the conversion of CO/H₂ to methanol

A 25 mL flame-dried Schlenk tube was charged with 0.1 mmol of Mn-PNY (or Mn-PNP, Mn-PNNP, Ru-PNS, Ru-PNNS), 2.5 mmol of base (KOMe, NaOMe, KOtBu, NaOtBu, sodium alcoholates of solvent, potassium alcoholates of solvent), 2.5 mmol of promoter (MeOH or solvent in the case of for example 2-ethylhexanol or 1-octanol) and 20 mL of dry, degassed 2-ethyl-1-hexanol. The mixture was stirred for 10 minutes before being transferred to a 100 mL autoclave. The autoclave was purged with carbon monoxide 3 times (5-7 bar) and then pressurized to 6 bar, then was filled with hydrogen to 30 bar overall pressure. The autoclave was heated to 120 °C and stirred at 1000 rpm for 22 h and the total pressure adjusted to 40 bar, which was maintained throughout the reaction. Then the reaction was placed in ice, cooled below 10 °C and depressurized. The reaction mixture was analyzed via GCMS analysis, the amount of methanol was determined via GC using n-decane as a standard (corrected for the amount of MeOH used as the promoter where applicable).

## Claims

1. Process for catalyzing a production of methanol from hydrogen and carbon monoxide taking place in the presence of at least one nucleophilic promoter, whereby a complex is used which comprises a transition metal (M) and at least one ligand designed as a chelating agent that binds tightly to three or more sites with two donor atoms which are connected via a ligand backbone with at least one Lewis base centrum (LB), **characterized in that** either both donor atoms are phosphor atoms whereby each donor atom has two identical substituents R' or R" and whereby the substituents R' and R" differ from each other (case 1) or that at least one donor atom is a phosphor atom being a chiral center with two different substituents R' and R" (case 2).

2. Process according to claim 1, **characterized in that** the transition metal (M) is selected of the group comprising Mn, Re, Cr, Mo, W, Fe, Ru, Co, Rh, Ir and Pd and/or the Lewis base centrum (LB) is an N atom of an amid or an NH-group.

3. Process according to claim 1 or 2, **characterized in that** the complex is described as

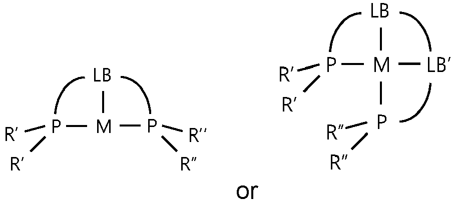

4. Process according to claim 1 or 2, **characterized in that** the complex is described as 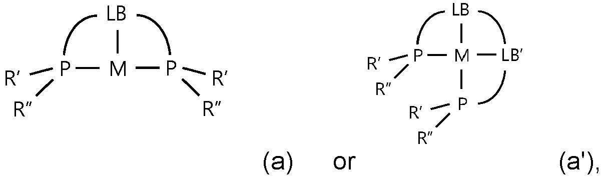 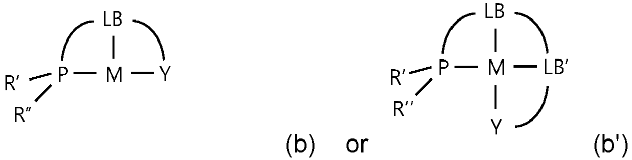 whereby Y is defined as a P, N, S or C atom being bound to the Lewis base centrum (LB) and featuring further substituents such that P is specified as PR‴Rʺʺ with R‴ and/or Rʺʺ as Et, nPr, iPr, nBu, iBu, tBu, Ph, Ph* [substituted], Naph or Naph* [substituted], N is specified as NH₂, NEt₂, N(nBu)₂, pyridine, pyrrole, indole, isoindole, imidazol, benzimidazol or aniline, S is specified as SMe, SEt, SPh, SPh* [substituted], SNaph or SNaph [substituted], and C is specified an N-heterocyclic carbene, aryl cyclopentadienyl or a derivate of cyclopentadienyl.

5. Process according to any of the previous claims, **characterized in that** R' and R" an alkyl or an aryl group, preferably are selected from a group comprising ethyl, n-propyl, isopropyl, n-butyl, isobutyl, t-butyl, phenyl, cyclohexyl, naphthyl as well as substituted phenyls, naphthyls and cyclohexyls and whereby R' and R" are two different substituents.

6. Process according to any of the previous claims, **characterized in that** the complex is selected from a group comprising 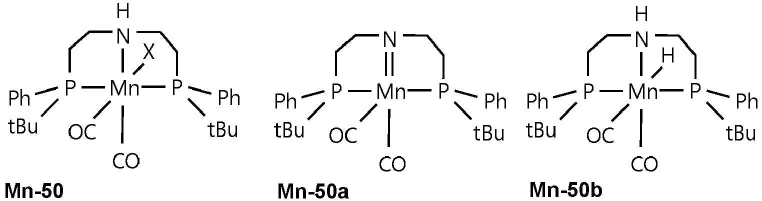 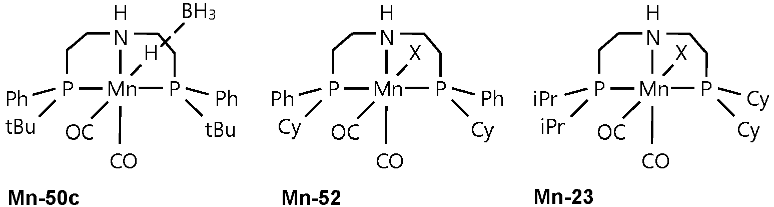 with X as F, Cl, Br, OH or an alcoholate.

7. Process according to any of the previous claims, **characterized in that** the process takes place in presence of a base.

8. Process according to claim 7, **characterized in that** the base is a hydroxide and/or an alkoxide and/or that the base is a methoxide salt, tert-butoxide salt or alcoholate of the utilized solvent and/or that the cation of the base is at least one of Li, Na, K, Rb, Mg, Ca, Sr or B, Al, Ti, Zr, Fe, Mn, Co or Mo.

9. Process according to any of the previous claims, **characterized in that** the promoter contains nitrogen and/or oxygen.

10. Process according to any of the previous claims, **characterized in that** the process takes place in a non-polar or polar solvent or solvent mixtures, particularly decaline, 1-octanol and/or 2-ethyl-1-hexanol.

11. Process according to any of the previous claims, **characterized in that** the solvent a primary or secondary alcohol and/or that the solvent acts also as the promoter.

12. Process according to any of claims 6 to 11, **characterized in that** the ratio r1 between base and catalyst is between 1:1 and 100:1 and/or that the ratio r2 of the promoter and base should be between 1:1 and 1:100.

13. Process according to any of the previous claims, **characterized in that** the process is run at a temperature below between 50 and 200 °C and/or that hydrogen as well as carbon monoxide are introduced with a partial pressure between 0.1 and 50 bar.

14. Process according to any of the previous claims, **characterized in that** the reaction setup runs as a semi-batch or continuous process.

15. Process according to any of the previous claims, **characterized in that** the process is run at total pressures between 40 and 100 bar and at a temperature between 80 and 150 °C, that the complex has the structural formula 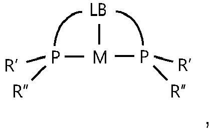 in particular 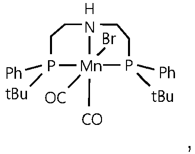 that the base is KOMe and/or K-(2-ethyl-1-hexanoate) and that the solvent and the promotor is 1-octanol and/or 2-ethyl-1-hexanol.
